# EUROPÄISCHE PATENTSCHRIFT

(11) **EP 2 451 361 B1**
(45) Veröffentlichungstag und Bekanntmachung des Hinweises auf die Patenterteilung: **06.11.2013**
(21) Anmeldenummer: 10724243.0
(22) Anmeldetag: 15.06.2010
(51) Int. Cl.: A61B 17/00

(54) **OKKLUDER**
OCCLUDER
OCCLUSEUR

(30) Priorität: 10.07.2009 CH 10732009; 22.01.2010 CH 862010
(43) Veröffentlichungstag der Anmeldung: 16.05.2012
(73) Patentinhaber: Carag AG, 6340 Baar (CH)
(72) Erfinder: STOOP, Hans, CH-4442 Diepflingen (CH); HÄFELFINGER, Mischa, CH-4460 Gelterkinden (CH)
(74) Vertreter: Clerc, Natalia
(86) Internationale Anmeldenummer: PCT/CH2010/000155
(87) Internationale Veröffentlichungsnummer: WO 2011/003213

(56) Entgegenhaltungen:
- WO-A1-03/061481
- WO-A1-2005/074813
- WO-A1-2005/110240
- WO-A1-2007/115117
- WO-A2-2006/041612
- US-A1- 2007 129 755
- US-A1- 2008 249 562

## Beschreibung

### TECHNISCHES GEBIET

Die vorliegende Erfindung betrifft einen Okkluder gemäss Oberbegriff des Patentanspruchs 1.

### STAND DER TECHNIK

Okkluder sind Implantate, die zum Verschliessen von Durchgängen oder Öffnungen in Kreislaufsystemen, wie beispielsweise Blutgefässen oder Shuntverbindungen eingesetzt werden und üblicherweise über eine in eine Vene eingeführte Schleuse positioniert und expandiert werden. Okkluder dienen insbesondere zum Verschluss von Öffnungen im Herzen oder in einem anderen Körperkanal des menschlichen oder tierischen Körpers. Sie dienen beispielsweise zum Verschluss eines persistierenden Ductus arteriosus (PDA), eines Vorhofseptumdefekts (ASD) oder eines Ventrikelseptumdefekts (VSD). Andere Anwendungen im menschlichen und tierischen Körper sind möglich.

Im Stand der Technik sind die unterschiedlichsten Ausgestaltungsformen von Okkludern bekannt. Sie können beispielsweise als Spiralfeder ausgebildet sein oder sich wie ein Regenschirm aufspannen lassen. Sie können ferner den Durchgang nur von einer Seite oder von beiden Seiten verschliessen. Diese Okkluder lassen sich üblicherweise in eine langgestreckte Form bringen, damit sie mittels des Katheters zum Durchgang, welcher verschlossen werden soll, gebracht werden können. Dort wird der Okkluder freigesetzt und er nimmt entweder selbsttätig oder geführt seine expandierte Gebrauchsform ein.

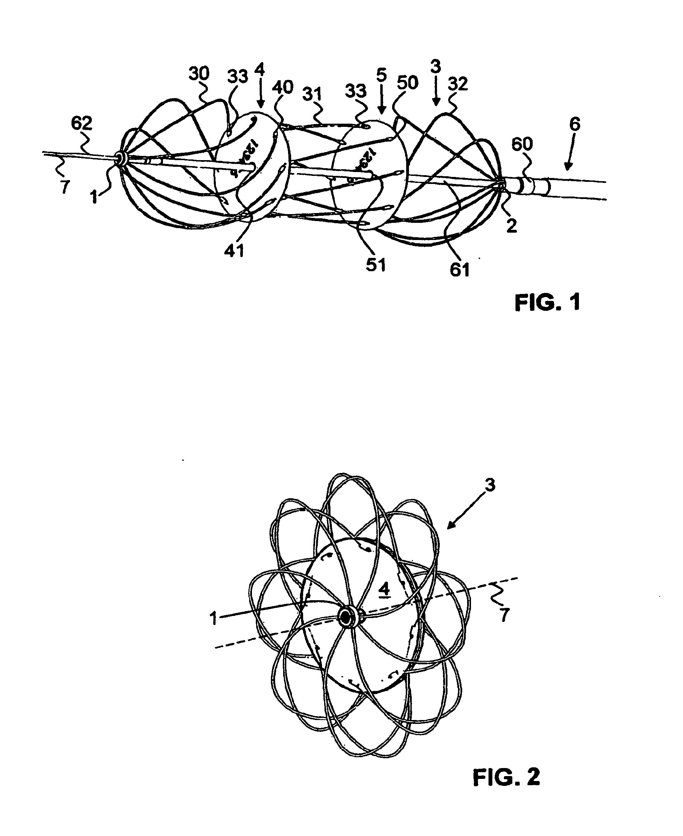

WO 2005/074813 offenbart einen Okkluder, entsprechend dem Oberbegriff das Anspruchs 1, mit zwei Expansionseinheiten und zwei Verschlusskörpern, welche auf je einer Seite des Durchgangs anliegen. Dieser Okkluder ist in den Figuren 1 und 2 dargestellt. Die Expansionseinheiten sind durch Drähte 3 gebildet, welche mit einem ersten Ende in einem ersten Kopplungsteil 1 und mit einem zweiten Ende in einem zweiten Kopplungsteil 2 gehalten sind. Durch Annäherung der zwei Kopplungsteile 1, 2 aneinander werden die Drähte 3 umgebogen und bilden zwei rosettenartige flache Strukturen. Die Verschlusskörper sind kreisförmige Membranen 4, 5, welche von den Drähten 3 durchsetzt sind. Beim Zusammenfühen der Kopplungsteile 1, 2 werden diese Membranen 4, 5 von den Drähten 3 aufgespannt. Sie verschliessen den Körperdurchgang von beiden Seiten. Die Kopplungsteile 1, 2 lassen sich in der expandierten Erscheinungsform gemäss Figur 2 ineinander stecken, so dass der Okkluder in dieser Erscheinungsform fixiert ist.

Aus WO 2007/115117 ist ein Okkluder mit beidseitig am Körperdurchgang anliegenden Expansionseinheiten bekannt, wobei der Abstand zwischen den Expansionseinheiten an die Tiefe des Durchgangs anpassbar ist.

### DARSTELLUNG DER ERFINDUNG

Es ist eine Aufgabe der Erfindung, einen verbesserten Okkluder zu schaffen, welcher eine Anpassung an die Tiefe der zu verschliessenden Öffnung respektive an die Breite der sie umgebenden Körperwandung ermöglicht

Diese Aufgabe löst ein Okkluder mit den Merkmalen des Patentanspruchs 1.

Der erfindungsgemässe Okkluder zum Verschliessen einer Öffnung in einem Kreislaufsystem ist von einer kompakten, sich entlang einer Längsachse erstreckenden Erscheinungsform in eine expandierte, sich quer zur Längsachse erstreckenden Erscheinungsform überführbar. Der Okkluder weist eine erste Expansionseinheit und eine zweite Expansionseinheit auf, welche in der expandierten Erscheinungsform auf je einer Seite der Öffnung anliegen. Der Okkluder weist ferner ein erstes Kopplungsteil und ein zweites Kopplungsteil auf, welche in Eingriff miteinander bringbar sind, so dass sie den Okkluder in seiner expandierten Erscheinungsform fixieren. Das erste Kopplungsteil weist einen Bereich auf, welcher in der expandierten Erscheinungsform mindestens teilweise zwischen der ersten und zweiten Expansionseinheit verläuft. Erfindungsgemäss ist dieser Bereich entlang, d.h. in Richtung, der Längsachse dehnbar ausgebildet. Vorzugsweise ist er entgegen einer Rückstellkraft dehnbar, also federnd oder elastisch ausgebildet.

Dank diesem dehnbaren mittleren Bereich des Okkluders lässt sich der Abstand zwischen den zwei Expansionseinheiten und, falls vorhanden, zwischen zwei Verschlusskörpern, selbsttätig einstellen. Ist der Okkluder in der zu verschliessenden Körperöffnung platziert und in seine expandierte Erscheinungsform gebracht und sind die zwei Kopplungsteile miteinander verbunden, so bestimmt die Tiefe der Öffnung bzw. die Dicke der sie umgebenden Körperwand den Abstand zwischen den zwei Expansionseinheiten. Der dehnbare Bereich wird entsprechend gedehnt, so dass sich die Expansionseinheiten und falls vorhanden, die Verschlusskörper, an die Wand anpassen und möglichst flach anliegen können. Die Öffnung wird dadurch optimal verschlossen und die als Fixierungsmittel wirkenden Expansionseinheiten liegen optimal an der Wand an.

Der dehnbare Bereich kann am distalen und/oder am proximalen Kopplungsteil vorgesehen sein. Proximal bedeutet hier dem Arzt und somit der Eintrittsstelle in den Körper zugewandt und distal dem Arzt abgewandt, also patientenseitig.

Die Expansionseinheit kann selber eine Verschlusseinheit zum Verschliessen der Öffnung bilden oder sie kann einen separaten Verschlusskörper aufspannen.

In einem bevorzugten Ausführungsbeispiel ist der Bereich zylinderförmig ausgebildet. Er kann eine Spiralfeder sein oder durch eine Gitterstruktur gebildet sein.

Vorzugsweise weist das diesen Bereich aufweisende erste Kopplungsteil einen zylinderförmigen Hohlschaft auf, welcher steckbar mit dem zweiten Kopplungsteil verbindbar ist, wobei der Hohlschaft den dehnbaren Bereich umfasst. Andere Arten von Verbindungen zwischen erstem und zweitem Kopplungsteil sind jedoch möglich. Beispielsweise kann eine Gewindeverbindung vorhanden sein.

Die Kopplungsteile sind vorzugsweise aus Metall oder Kunststoff oder Kombinationen davon gefertigt. Vorzugsweise ist der dehnbare Bereich aus Metall gefertigt.

Vorzugsweise weist der Okkluder einen Aufbau auf, wie er in der eingangs erwähnten WO 2005/074813 beschrieben ist, wobei der Hohlschaft des einen Kopplungsteils bzw. der einen Halterung dehnbar ausgebildet ist. Andere Ausgestaltungsformen von Okkludern können jedoch ebenfalls erfindungsgemäss mit dehnbaren Kopplungsteilen versehen sein.

In einer bevorzugten Ausführungsform ist die erste Expansionseinheit am ersten Kopplungsteil und die zweite Expansionseinheit ist am zweiten Kopplungsteil befestigt. Vorzugsweise weisen die Expansionseinheiten Drähte auf, deren erste Enden in den Kopplungsteilen gehalten sind, wobei die Drähte jeder Expansionseinheit in der expandierten Erscheinungsform eine im wesentlichen flache Struktur bilden, welche auf je einer Seite der Öffnung anliegt.

Vorzugsweise weist mindestens eine der zwei Expansionseinheiten einen Verschlusskörper auf oder sie bildet selber den Verschlusskörper. Vorzugsweise ist der Verschlusskörper eine Membran, welche von den Drähten durchsetzt ist. Die Membran kann kreisförmig ausgestaltet sein oder eine andere Form aufweisen. Sie lässt sich vorzugsweise mittels der Drähte in die expandierte Erscheinungsform aufspannen, beispielsweise indem diese durch äussere Krafteinwirkung in rosettenartige Expansionseinheiten umklappen

Vorzugsweise sind die zwei Expansionseinheiten und falls vorhanden, die Membranen, spiegelsymmetrisch zueinander ausgebildet.

In einer Ausführungsform weist das zweite Kopplungsteil einen zylindertörmigen Stutzen auf, welcher in den Hohlschaft des ersten Kopplungsteils einsteckbar ist, wobei der Stutzen an seinem freien Ende eine umlaufende Rückhaltewulst aufweist und wobei das erste Kopplungsteil eine im Innern des Hohlschaftes verlaufende Nut aufweist zwecks Verbindung mit der Rückhaltewulst. Diese Ausführungsform lässt sich insbesondere in einem Okkluder gemäss der eingangs beschriebenen WO 2005/074813 oder einem anderen Okkluder verwenden, wobei das Kopplungsteil in einem Beispiel, welches nicht Teil der Erfindung ist, nicht zwingend einen dehnbaren Bereich aufweisen muss.

Weitere Ausführungsbeispiele sind in den abhängigen Ansprüchen beschrieben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: einen Okkluder gemäss dem Stand der Technik in einer Erscheinungsform, welche einen Übergang zwischen einer kompakten und einer expandierten Erscheinungsform darstellt;
- Figur 2: den Okkluder gemäss Figur 1 in der expandierten Erscheinungsform;
- Figur 3: einen Längsschnitt durch ein distales Kopplungsteil in einer ersten Ausführungsform;
- Figur 4: eine Seitenansicht des distalen Kopplungsteils gemäss Figur 3;
- Figur 5: eine Seitenansicht des distalen Kopplungsteils gemäss Figur 3 in entlang der Längsachse verkürzten Darstellung;
- Figur 6a: eine Seitenansicht eines distalen Kopplungsteils gemäss einer zweiten Ausführungsform in entlang der Längsachse verkürzten Darstellung;
- Figur 6b: eine weitere Seitenansicht des distalen Kopplungsteils gemäss Figur 6a;
- Figur 7a: eine Seitenansicht eines distalen Kopplungsteils gemäss einer dritten Ausführungsform in entlang der Längsachse verkürzten Darstellung;
- Figur 7b: eine weitere Seitenansicht des distalen Kopplungsteils gemäss Figur 7a;
- Figur 8: eine perspektivische Darstellung eines proximalen Kopplungsteils gemäss einer vierten Ausführungsform;
- Figur 9: eine perspektivische Darstellung eines distalen Kopplungsteils, welches dem proximalen Kopplungsteils gemäss Figur 8 zugehörig ist;
- Figur 10: einen Längsschnitt durch die Kopplungsteile gemäss den Figuren 8 und 9, welche miteinander im Eingriff stehen;
- Figur 11: eine schematische Darstellung eines in einer Öffnung platzierten Okkluders in einer ersten Anwendung und
- Figur 12: eine schematische Darstellung eines in einer Öffnung platzierten Okkluders in einer zweiten Anwendung.

### BESCHREIBUNG BEVORZUGTER AUSFÜHRUNGSFORMEN

In den Figuren 1 und 2 ist ein Okkluder gemäss dem Stand der Technik dargestellt. Dieser Okkluder lässt sich, wie nachfolgend beschrieben, mit einem erfindung Kopplungsteil versehen. Dieser Okkluder stellt eine bevorzugte Ausführungsform dar. Die erfindungsgemässe Lehre lässt sich jedoch auch auf andere Okkluder anwenden.

Der Okkluder weist ein distales Kopplungsteil 1 und ein proximales Kopplungsteil 2 auf. Die Kopplungsteile 1, 2 sind vorzugsweise aus Kunststoff oder Metall oder aus Kombinationen davon gefertigt. Der nachfolgend beschriebene mittlere Bereich ist vorzugsweise aus Metall gefertigt. Beispielsweise besteht er aus Phynox oder Nitinol.

Zwischen diesen zwei Kopplungsteilen 1, 2 verlaufen Drähte 3, welche mit ihren ersten Enden im distalen Kopplungsteil 1 und mit ihren zweiten Enden im proximalen Kopplungsteil 2 gehalten sind. Die Drähte 3 sowie die nachfolgend beschriebenen Hülsen 33 bestehen vorzugsweise aus Platiniridium oder Nitinol oder einem resorbierbaren Material.

Der Okkluder umfasst ferner zwei Membrane 4, 5, welche jeweils eine zentrale Durchgangsöffnung 41, 51 und gleichmässig über ihren Umfang verteilte periphere Durchgangsöffnungen 40, 50 aufweisen. Die Membranen 4, 5 sind vorzugsweise aus PET oder Dacron gefertigt.

Die peripheren Durchgangsöffnungen 40, 50 sind von den Drähten 3 durchsetzt. Dadurch sind die einzelnen Drähte 3 in einen distalen Drahtabschnitt 30, einen zwischen den zwei Membranen 4,5 liegenden mittleren Drahtabschnitt 31 und einen proximalen Drahtabschnitt 32 unterteilt. Die Drähte 3 sind mit unverschiebbar auf den Drähten gehaltenen Hülsen versehen, welche die relative Position der Membranen 4, 5 zu diesen Abschnitten 30, 31, 32 definieren. Auf die Hülsen kann auch verzichtet werden bzw. sie können durch ähnlich wirkende Mittel ersetzt werden.

Die zentralen Durchgangsöffnungen 41, 51 weisen einen so grossen Durchmesser auf, dass sie vom distalen und/oder proximalen Kopplungsteil 1, 2 durchsetzt werden können. Dabei weisen die zwei Kopplungsteile 1, 2 je einen Flansch 11, 21 auf, dessen Aussendurchmesser vorzugsweise grösser ist als der Durchmesser der Durchgangsöffnungen 41, 51.

In Figur 1 ist erkennbar, wie der Okkluder in eine Körperöffnung einführbar ist. Der Okkluder weist eine komprimierte Erscheinungsform auf. Diese ist in Figur 1 nicht wiedergegeben. In der komprimierten Erscheinungsform ist der Okkluder langgezogen und die Drähte 3 sind annähernd vollständig gestreckt. Dadurch sind die Membranen 4, 5 zusammengeknüllt bzw. zusammengefaltet.

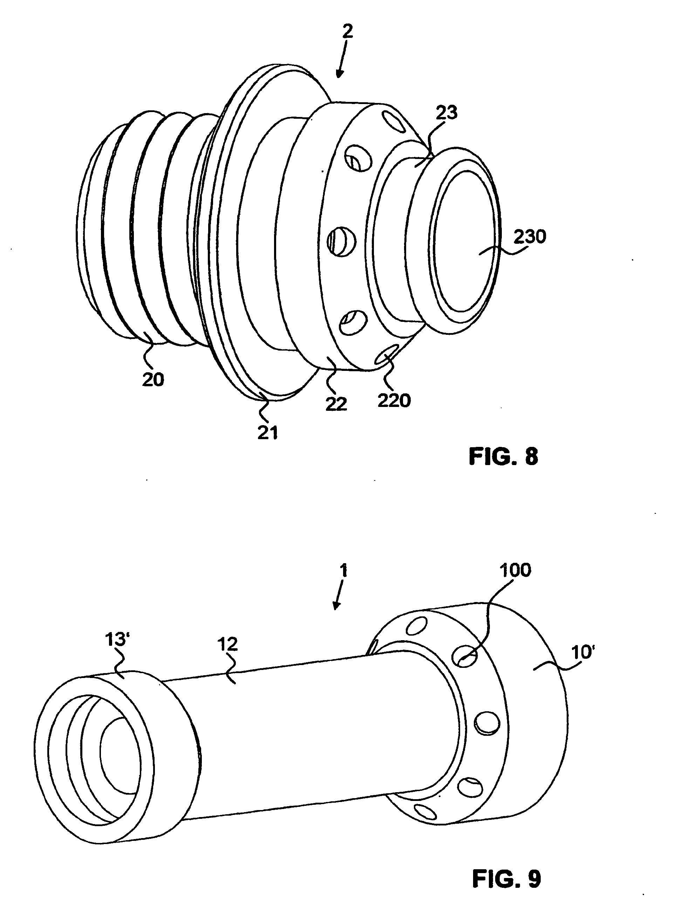

Der Okkluder lässt sich mittels eines Katheters 6 an die Stelle der zu verschliessenden Öffnung bringen. Dieser Katheter 6 ist in Figur 1 nur mit seinem distalen Ende wiedergegeben. Er weist einen hohlen Katheterkörper 60 auf, welcher vorzugsweise von einem flexiblen Schlauch gebildet ist. Auf diesem Katheterkörper 60 ist an seinem Ende ein steifer Kopf mit einem Innengewinde aufgesteckt. Mit diesem Innengewinde wird der Katheter 6 mit einem Aussengewinde 20 (siehe Figur 8) des proximalen Kopplungsteils 2 des Okkluders verbunden.

Im Katheterkörper 60 verläuft ein Katheterschlauch 61, welcher den proximalen Kopplungsteil 2 durchsetzt und welche in eine Katheterspitze übergeht. Der Katheterschlauch ist vorzugsweise flexibel ausgebildet, wobei er über seine Länge verschiedene Härten äufweisen kann und insbesondere auch einen steifen Bereich aufweisen kann. Diese Katheterspitze wird mit dem distalen Kopplungsteil 1 verbunden. Ein Führungsdraht 62, welcher den Katheterschlauch 61 durchsetzt, erleichtert das Einführen des komprimierten Okkluders.

Der Okkluder wird komprimiert und in seine expandierte Erscheinungsform gebracht, indem eine über das Kathetersystem übertragene axiale Zugkraft auf die Drähte 3 aufgebracht wird. Durch Zurückziehen des distalen Endes des Schlauchs 61 zum Katheterkörper 60 hin, wird der Okkluder aufgespannt und expandiert. Dabei bilden die Drähte 3 eine proximale und eine distale Expansionseinheit und die Membranen 4, 5 einen distalen und einen proximalen Verschlusskörper. Alternativ oder zusätzlich kann der Okkluder auch in seine expandierte Form gebracht werden, indem das proximale Kopplungsteil 2 mittels des Katheterkörpers 60 zum distalen Kopplungsteil 1 hin gestossen wird und der Okkluder so komprimiert wird. Diese Einheiten und diese Verschlusskörper kommen auf je einer Seite des zu verschliessenden Durchgangs zu liegen. Die Drähte 3 bilden dabei annähernd flache Strukturen. Beim Zusammenfüren sind sie zu zwei rosettenartigen oder blütentörmigen Gebilden umgeklappt worden. Sie kippen also in diese Gebilde um (to twist).

Ein derartiger expandierter Zustand ist in Figur 2 dargestellt. Die distalen Drahtabschnitte 31 der Drähte 3 bilden eine erste Expansionseinheit und die proximalen Drahtabschnitte 32 der Drähte 3 bilden eine zweite Expansionseinheit. Diese zwei Expansionseinheiten spannen die Membranen 4, 5 auf und fixieren den Okkluder auf beiden Seiten eines Durchgangs durch eine Körperwand.

Ist der Okkluder im Durchgang platziert, so wird der Schlauch 61 noch weiter zurückgezogen bzw. der Katheterkörper 60 noch weiter vorgestossen, so dass die zwei Kopplungsteile 1, 2 ineinander greifen und den Okkluder in der aufgespannten Position fixieren. Die Verbindungen zwischen Katheter und Okkluder werden gelöst und der Katheter wird entfernt.

Erfindungsgemäss ist nun mindestens eines der zwei Kopplungsteile mit einem dehnbaren Bereich ausgestattet, wobei mindestens ein Teil dieses Bereichs im expandierten Zustand des Okkluders zwischen den zwei Expansionseinheiten 30, 32 zu liegen kommt. In diesem Beispiel ist das distale Kopplungsteil 1 mit einem derartigen Bereich 12 versehen.

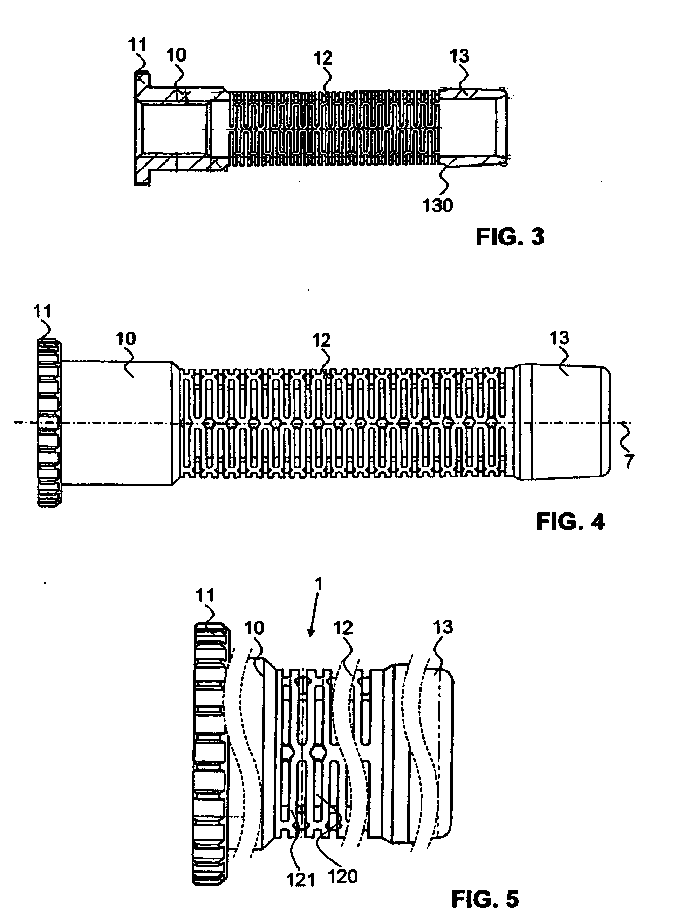

In den Figuren 3, 4 und 5 ist ein erstes Ausführungsbeispiel eines derartigen distalen Kopplungsteils 1 dargestellt, welches einen Hohlschaft aufweist. Die Grundform des Kopplungsteils 1 ist zylinderförmig und es ist durchgehend hohl mit einer Eingangsöffnung und einer Ausgangsöffnung ausgebildet. Im Bereich der proximalen Eingangsöffnung ist vorzugsweise ein Innengewinde zur Verbindung mit dem Katheterschlauch 61 vorhanden.

Das Kopplungsteil 1 weist ein distales Endstück 10 mit einem Endflansch 11 auf. Der Endflansch 11 weist einen grösseren Aussendurchmesser auf als die zentrale Durchgangsöffnung 41 der distalen Membran 4.

In einer Ausführungsform sind im Endstück 10 bzw. im Endflansch 11 hier nicht dargestellte Aufnahmeöffnungen vorhanden, um die Enden der Drähte 3 aufzunehmen und zu halten. An das Endstück 10 schliesst der dehnbare Bereich 12 an. Er weist vorzugsweise einen gleichbleibenden äusseren Durchmesser auf. Dieser äussere Durchmesser ist gleich oder kleiner als der Durchmesser der zentralen Durchgangsöffnung 41 der distalen Membran 4 und vorzugsweise ebenfalls der proximalen Membran 5.

Der dehnbare Bereich 12 ist, wie in Figur 5 erkennbar ist, mit einer Gitterstruktur versehen. Stege 121 werden von Schlitzen 120 abgelöst. Diese Gitterstruktur kann sich entlang der Längsachse des Okkluders bzw. entlang der Längsachse des distalen Kopplungsteils 1 dehnen, so dass durch äussere Krafteinwirkung, d.h. durch die Wand der zu verschliessenden Öffnung, das distale Kopplungsteil 1 verlängert wird. Die Längsachse des Okkluders und des distalen Kopplungsteils verlaufen deckungsgleich zueinander und sie sind in den Figuren mit der Bezugsziffer 7 versehen.

An den dehnbaren Bereich 12 schliesst ein Einsteckstutzen 13 an, welcher mit einer umlaufenden Rückhaltenase 130 versehen ist. Dieser Einsteckstutzen 13 lässt sich in einen entsprechenden Aufnahmestutzen des zugehörigen proximalen Kopplungsteils 2 einstecken und mittels der Rückhaltenase 130 in einer entsprechenden inneren Nut des proximalen Kopplungsteils 2 fixieren.

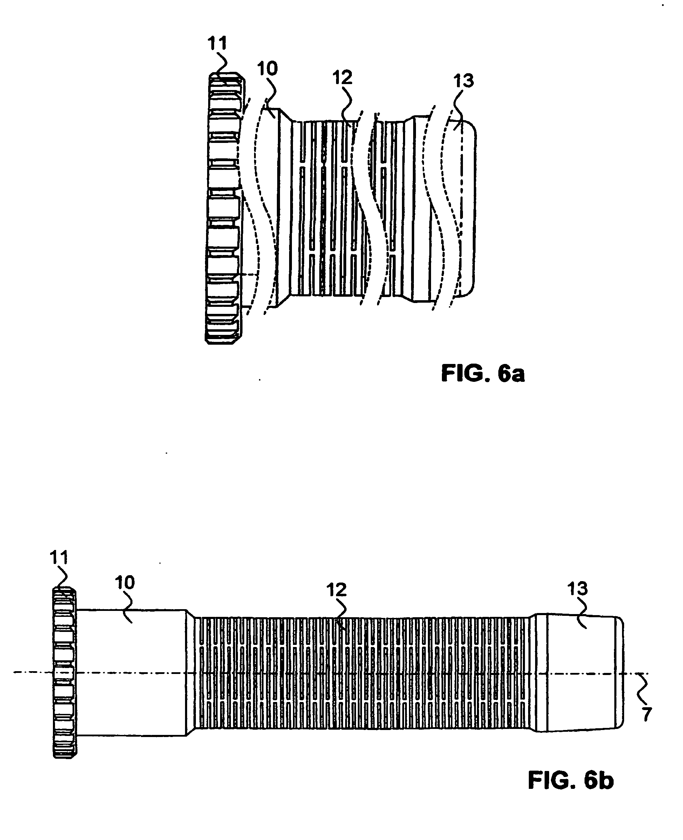

In den Figuren 6a und 6b ist ein zweites Ausführungsbeispiel dargestellt. Das distale Kopplungsteil ist im Wesentlichen gleich ausgebildet wie im oben beschriebenen Beispiel. Die Gitterstruktur des dehnbaren Bereichs 12 weist jedoch eine andere Form auf. Die Stege und Schlitze sind schmaler ausgebildet als im ersten Beispiel.

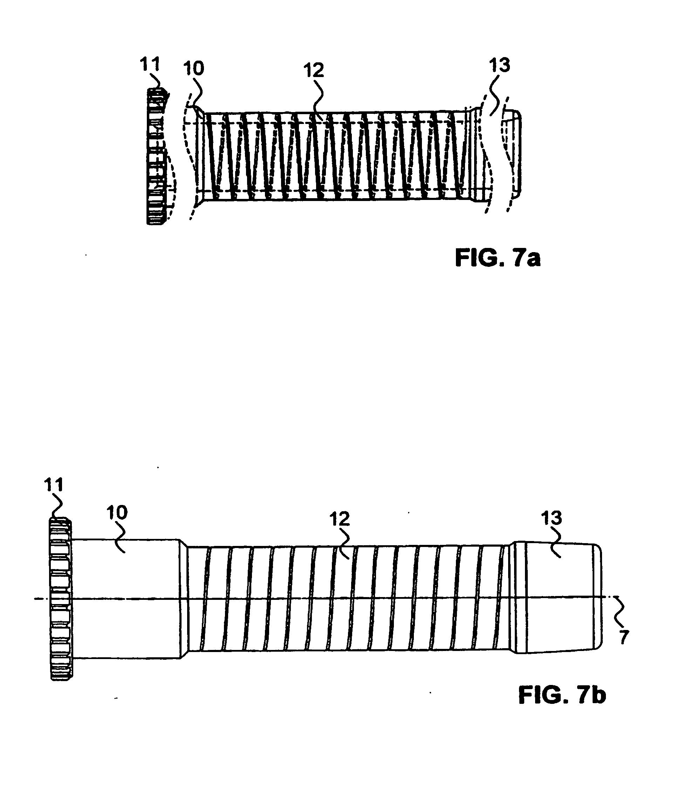

In der dritten Ausführungsform gemäss den Figuren 7a und 7b ist der dehnbaren Bereich 12 als Spiralfeder ausgebildet, bzw. der dehnbare Bereich 12 ist mit einem helixartig umlaufenden Schlitz versehen.

In der Ausfürungsform gemäss den Figuren 8 bis 10 wird das proximale Kopplungsteil 2 in das distale Kopplungsteil 1 eingesteckt. Das proximale Kopplungsteil 2 gemäss Figur 8 weist einen Gewindestutzen 20 mit einem Aussengewinde zur Verbindung mit dem Katheterkörper 60 auf. An diesen Stutzen 20 schliesst der Flansch 21 an, welcher einen grösseren Aussendurchmesser aufweist als die zentrale Durchgangsöffnung 51 der proximalen Membran 5. Anschliessend folgt der Aufnahmekörper 22 mit den Aufnahmeöffnungen 220, in welchen die Enden der Drähte 3 gehalten sind Ein Einsteckstutzen 23 mit einer umlaufenden Rückhaltewulst 230 bildet das distale Ende dieses Kopplungsteils 2.

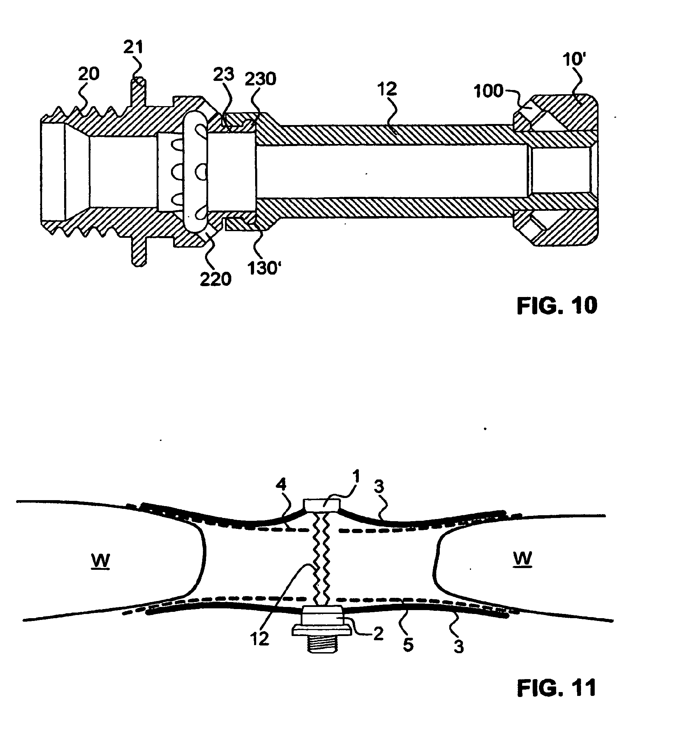

Das Gegenstück dazu ist in Figur 9 dargestellt. Hier weist das distale Kopplungsteil 1 ein bereits als Flansch ausgebildetes distales Endstück 10' mit Aufnahmeöffnungen 100 auf. Das proximale Ende dieses Kopplungsteils 1 ist durch einen Aufnahmestutzen 13' gebildet, in welchen der Einsteckstutzen 23 einsteckbar ist. Im Aufnahmestutzen 13' ist eine Nut 130' vorhanden, in welche die Rückhaltewulst 230 eingreift, wie dies in Figur 10 erkennbar ist. Die Kopplung kann alternativ auch gleich erfolgen wie die Kopplung gemäss den in den Figuren 3, 4 und 6b dargestellten Ausführungsformen.

Auch die Ausführungsform gemäss den Figuren 8 bis 10 weist einen dehnbaren Bereich 12 auf, welcher gleich ausgebildet sein kann wie in den oben beschriebenen Beispielen.

Des weiteren ist in Figur 10 erkennbar, dass distale Kopplungsteil 1 zweiteilig ausgebildet ist. Der Grundkörper, welcher den dehnbaren Bereich 12 umfasst, ist vorzugsweise aus Metall oder Kunststoff gefertigt. Dieser Grundkörper 12 ist an einem Ende von einer Hülse 10' umgeben, welche das Endstück des distalen Kopplungsteils 1 bildet. In diesem Endstück 10' sind Aufnahmeöffnungen 100 zur Aufnahme der Drähte 3 vorhanden. Die Hülse 10' ist vorzugsweise über das distale Ende des dehnbaren Bereichs 12 geschoben und dort insbesondere formschlüssig gehalten sein. Der dehnbare Bereich 12 kann an diesem distalen Ende einen radial nach aussen vorstehenden Flansch aufweisen, welcher in eine entsprechende, innere umlaufende Nut oder Absatz der Hülse 10' einschnappen kann. Dies ist hier nicht dargestellt. Vorzugsweise ist das distale Endstück 10' sowie das proximale Kopplungsteil 2 vorzugsweise aus Kunststoff hergestellt. Diese zweiteilige Ausbildung und die genannte Materialwahl lässt sich auch auf die anderen hier beschriebenen Ausführungsbeispiele anwenden.

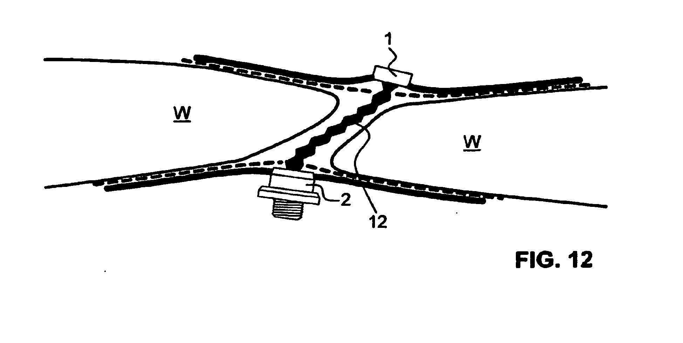

In den Figuren 11 und 12 ist der Okkluder in einer zu verschliessenden Körperöffnung platziert. Die rosettenartig umgebogenen Drähte 3 und die Membranen 4, 5 liegen auf beiden Seiten der Körperwand W an. Die Kopplungsteile 1, 2 sind im Eingriff miteinander gebracht, vorzugsweise ineinander gesteckt. Es ist erkennbar, wie sich der zwischen den Expansionseinheiten 3 und zwischen den Membranen 4, 5 liegende dehnbare Bereich 12 den äusserlichen Gegebenheiten anpasst und sich ausdehnt, so dass die Expansionseinheiten 3 und die Membranen 4,5 möglichst flach beidseitig an der Wand W anliegen können.

Der erfindungsgemässe Okkluder erlaubt somit eine selbsttätige Einstellung des Abstandes zwischen zwei Expansions- und/oder Verschlusseinheiten nach Massgabe der strukturellen Gegebenheiten am Ort der zu verschliessenden Öffnung.

### BEZUGSZEICHENLISTE

| | | | |
|---|---|---|---|
| 1 | distales Kopplungsteil | 30 | distaler Drahtabschnitt |
| 10 | distales Endstück | 31 | mittlerer Drahtabschnitt |
| 10' | distales Endstück | 32 | proximaler Drahtabschnitt |
| 100 | Aufnahmeöffnung | 33 | Hülse |
| 11 | Endflansch | | |
| 12 | dehnbarer Bereich | 4 | distale Membran |
| 120 | Schlitz | 40 | periphere Durchgangsöffnung |
| 121 | Steg | 41 | zentrale Durchgangsöffnung |
| 13 | Einsteckstutzen | | |
| 13' | Aufnahmestutzen | 5 | proximale Membran |
| 130 | Rückhaltenase | 50 | periphere Durchgangsöffnung |
| 130' | Nut | 51 | zentrale Durchgangsöffnung |
| | | | |
| 2 | proximales Kopplungsteil | 6 | Katheter |
| 20 | Gewindestutzen | 60 | Katheterkörper |
| 21 | Flansch | 61 | Katheterschlauch |
| 22 | Aufnahmekörper | 62 | Führungsdraht |
| 220 | Aufnahmeöffnung | | |
| 23 | Einsteckstutzen | 7 | Längsachse |
| 230 | Rückhaltewulst | | |
| | | W | Wand |
| 3 | Draht | | |

## Patentansprüche

1. Okkluder zum Verschliessen einer Öffnung in einem Kreislaufsystem, wobei der Okkluder von einer kompakten, sich entlang einer Längsachse (7) erstreckenden Erscheinungsform in eine expandierte Erscheinungsform überführbar ist, wobei der Okkluder eine erste Expansionseinheit und eine zweite Expansionseinheit aufweist, welche in der expandierten Erscheinungsform auf je einer Seite der Öffnung anliegen, und wobei der Okkluder ferner ein erstes Kopplungsteil (1) und ein zweites Kopplungsteil (2) aufweist, welche in Eingriff miteinander bringbar sind, so dass sie den Okkluder in seiner expandierten Erscheinungsform fixieren, wobei das erste Kopplungsteil (1) einen Bereich (12) aufweist, welcher in der expandierten Erscheinungsform mindestens teilweise zwischen der ersten und zweiten Expansionseinheit verläuft, **dadurch gekennzeichnet, dass** dieser Bereich (12) entlang der Längsachse (7) dehnbar ausgebildet ist.

2. Okkluder nach Anspruch 1, wobei dieser Bereich (12) federnd ausgebildet ist.

3. Okkluder nach einem der Ansprüche 1 oder 2, wobei dieser Bereich (12) zylinderförmig ausgebildet ist.

4. Okkluder nach einem der Ansprüche 1 bis 3, wobei dieser Bereich (12) durch eine Spiralfeder gebildet ist.

5. Okkluder nach einem der Ansprüche 1 bis 3, wobei dieser Bereich (12) durch eine Gitterstruktur gebildet ist.

6. Okkluder nach einem der Ansprüche 1 bis 5, wobei das diesen Bereich (12) aufweisende erste Kopplungsteil (1) einen zylinderförmigen Hohlschaft aufweist, welcher steckbar mit dem zweiten Kopplungsteil (2) verbindbar ist, wobei der Hohlschaft den dehnbaren Bereich (12) umfasst.

7. Okkluder nach einem der Ansprüche 1 bis 6, wobei der dehnbare Bereich (12) aus Metall gefertigt ist.

8. Okkluder nach einem der Ansprüche 1 bis 7, wobei die erste Expansionseinheit am ersten Kopplungsteil (1) und die zweite Expansionseinheit am zweiten Kopplungsteil (2) befestigt ist.

9. Okkluder nach Anspruch 8, wobei die Expansionseinheiten Drähte (3) aufweisen, deren Enden in den Kopplungsteilen (1, 2) gehalten sind, wobei die Drähte (3) jeder Expansionseinheit in der expandierten Erscheinungsform eine im wesentlichen flache Struktur bilden, welche auf je einer Seite der Öffnung anliegt.

10. Okkluder nach einem der Ansprüche 1 bis 9, wobei mindestens eine der zwei Expansionseinheiten einen Verschlusskörper (4, 5) aufweist.

11. Okkluder nach Anspruch 10, wobei der Verschlusskörper eine Membran (4, 5) ist, welche von den Drähten (3) durchsetzt ist.

12. Okkluder nach einem der Ansprüche 1 bis 11, wobei die zwei Expansionseinheiten spiegelsymmetrisch zueinander ausgebildet sind.

13. Okkluder nach den Ansprüchen 9 und 10, wobei der Verschlusskörper (4, 5) mittels der Drähte (3) der zugehörigen Expansionseinheit in die expandierte Erscheinungsform aufspannbar ist.

14. Okkluder nach einem der Ansprüche 9 oder 13, wobei die Drähte (3) mit ihrem ersten Ende im ersten Kopplungsteil (1) und mit ihrem zweiten Ende im zweiten Kopplungsteil (2) gehalten sind.

15. Okkluder nach Anspruch 6, wobei das zweite Kopplungsteil (2) einen zylinderförmigen Stutzen (23) aufweist, welcher in den Hohlschaft (13') des ersten Kopplungsteils (1) einsteckbar ist, wobei der Stutzen (23) an seinem freien Ende eine umlaufende Rückhaltewulst (230) aufweist und wobei das erste Kopplungsteil (1) eine im Innern des Hohlschaftes (13') verlaufende Nut (130') aufweist zwecks Verbindung mit der Rückhaltewulst (230).

## Claims

1. An occluder for occluding an opening in a circulatory system, wherein the occluder can be converted from a compact manifestation, extending along a longitudinal axis (7), into an expanded manifestation, wherein the occluder has a first expansion unit and a second expansion unit, which in each case rest against one side of the opening in the expanded manifestation, and wherein the occluder furthermore has a first coupling part (1) and a second coupling part (2), which can be brought to engage into one another such that they fix the occluder in the expanded manifestation thereof, wherein the first coupling part (1) has a region (12) that at least in part runs between the first and the second expansion unit in the expanded manifestation, **characterized in that** this region (12) is embodied such that it can stretch along the longitudinal axis (7).

2. The occluder as claimed in claim 1, wherein this region (12) has a resilient design.

3. The occluder as claimed in one of claims 1 or 2, wherein this region (12) has a cylindrical design.

4. The occluder as claimed in one of claims 1 to 3, wherein this region (12) is formed by a helical spring.

5. The occluder as claimed in one of claims 1 to 3, wherein this region (12) is formed by a grid structure.

6. The occluder as claimed in one of claims 1 to 5, wherein the first coupling part (1) having this region (12) has a cylindrical hollow shaft, which can be connected in a plug-in fashion to the second coupling part (2), wherein the hollow shaft comprises the stretchable region (12).

7. The occluder as claimed in one of claims 1 to 6, wherein the stretchable region (12) is made of metal.

8. The occluder as claimed in one of claims 1 to 7, wherein the first expansion unit is attached to the first coupling part (1) and the second expansion unit is attached to the second coupling part (2).

9. The occluder as claimed in claim 8, wherein the expansion units have wires (3), the ends of which are held in the coupling parts (1, 2), wherein the wires (3) of each expansion unit form a substantially planar structure in the expanded manifestation, which planar structure in each case rests against one side of the opening.

10. The occluder as claimed in one of claims 1 to 9, wherein at least one of the two expansion units has a closure body (4, 5).

11. The occluder as claimed in claim 10, wherein the closure body is a membrane (4, 5) that is penetrated by the wires (3).

12. The occluder as claimed in one of claims 1 to 11, wherein the two expansion units have a mirror symmetric design with respect to one another.

13. The occluder as claimed in claims 9 and 10, wherein the closure body (4, 5) can be opened into the expanded manifestation by means of the wires (3) of the associated expansion unit.

14. The occluder as claimed in one of claims 9 or 13, wherein the wires (3) are held in the first coupling part (1) with their first end and in the second coupling part (2) with their second end.

15. The occluder as claimed in claim 6, wherein the second coupling part (2) has a cylindrical port (23), which can be inserted into the hollow shaft (13') of the first coupling part (1), wherein the port (23) has an encircling retention bead (230) at the free end thereof and wherein the first coupling part (1) has a groove (130') running in the interior of the hollow shaft (13') for the purpose of connection to the retention bead (230).

## Revendications

1. Occluseur destiné à fermer une ouverture dans un système de circuit, dans lequel l'occluseur peut être converti d'un état compact, s'étendant le long d'un axe longitudinal (7), à un état expansé, dans lequel l'occluseur présente une première unité d'expansion et une deuxième unité d'expansion, qui s'appliquent dans l'état expansé chacune sur un côté de l'ouverture, et dans lequel l'occluseur présente en outre une première pièce de couplage (1) et une deuxième pièce de couplage (2), qui peuvent être mises en prise l'une avec l'autre, de telle manière qu'elles fixent l'occluseur dans son état expansé, dans lequel la première pièce de couplage (1) présente une zone (12) qui, dans l'état expansé, s'étend au moins en partie entre la première et la deuxième unités d'expansion, **caractérisé en ce que** cette zone (12) est réalisée sous forme extensible le long de l'axe longitudinal (7).

2. Occluseur selon la revendication 1, dans lequel cette zone (12) est réalisée sous forme élastique.

3. Occluseur selon l'une des revendications 1 ou 2, dans lequel cette zone (12) est de forme cylindrique.

4. Occluseur selon l'une quelconque des revendications 1 à 3, dans lequel cette zone (12) est formée par un ressort hélicoïdal.

5. Occluseur selon l'une quelconque des revendications 1 à 3, dans lequel cette zone (12) est formée par une structure de grille.

6. Occluseur selon l'une quelconque des revendications 1 à 5, dans lequel la première pièce de couplage (1) présentant cette zone (12) présente un arbre creux cylindrique, qui peut être assemblé par engagement avec la deuxième pièce de couplage (2), dans lequel l'arbre creux comprend la zone extensible (12).

7. Occluseur selon l'une quelconque des revendications 1 à 6, dans lequel la zone extensible (12) est fabriquée en métal.

8. Occluseur selon l'une quelconque des revendications 1 à 7, dans lequel la première unité d'expansion est fixée sur la première pièce de couplage (1) et la deuxième unité d'expansion est fixée sur la deuxième pièce de couplage (2).

9. Occluseur selon la revendication 8, dans lequel les unités d'expansion comprennent des fils (3), dont les extrémités sont maintenues dans les pièces de couplage (1, 2), dans lequel les fils (3) de chaque unité d'expansion forment, dans l'état expansé, une structure essentiellement plate, qui s'applique respectivement sur un côté de l'ouverture.

10. Occluseur selon l'une quelconque des revendications 1 à 9, dans lequel au moins une des deux unités d'expansion présente un corps de fermeture (4, 5) .

11. Occluseur selon la revendication 10, dans lequel le corps de fermeture est une membrane (4, 5), qui est traversée par les fils (3).

12. Occluseur selon l'une quelconque des revendications 1 à 11, dans lequel les deux unités d'expansion sont réalisées de façon miroir symétrique l'une par rapport à l'autre.

13. Occluseur selon les revendications 9 et 10, dans lequel le corps de fermeture (4, 5) peut être serré au moyen des fils (3) de l'unité d'expansion correspondante dans l'état expansé.

14. Occluseur selon l'une des revendications 9 ou 13, dans lequel les fils (3) sont maintenus par leur première extrémité dans la première pièce de couplage (1) et par leur deuxième extrémité dans la deuxième pièce de couplage (2).

15. Occluseur selon la revendication 6, dans lequel la deuxième pièce de couplage (2) présente un embout cylindrique (23), qui peut être engagé dans l'arbre creux (13') de la première pièce de couplage (1), dans lequel l'embout (23) présente un bourrelet de retenue périphérique (230) à son extrémité libre et dans lequel la première pièce de couplage (1) présente une rainure (130') s'étendant à l'intérieur de l'arbre creux (13') en vue de l'assemblage avec le bourrelet de retenue (230).
